# EUROPEAN PATENT SPECIFICATION

(11) **EP 2 313 115 B1**
(45) Date of publication and mention of the grant of the patent: **15.05.2019**
(21) Application number: 09769029.1
(22) Date of filing: 26.06.2009
(51) Int. Cl.: A61K 47/34, A61K 9/107

(54) **POLYMERIC DELIVERY SYSTEMS FOR ACTIVE AGENTS**
POLYMER-ABGABESYSTEME FÜR WIRKSTOFFE
SYSTÈMES D'ADMINISTRATION POLYMÈRE POUR AGENTS ACTIFS

(30) Priority: 26.06.2008 US 133154 P; 08.07.2008 US 134209 P
(43) Date of publication of application: 27.04.2011
(73) Proprietor: Jordan, Rainer, 01705 Freital (DE); Luxenhofer, Robert, 97084 Würzburg (DE); Kabanov, Alexander V., Chapel Hill, NC 27514 (US)
(72) Inventor: KABANOV, Alexander, V., Omaha NE 68154 (US); JORDAN, Rainer, 01219 Dresden (DE); LUXENHOFER, Robert, 01187 Dresden (DE)
(74) Representative: Vossius & Partner Patentanwälte Rechtsanwälte mbB
(86) International application number: PCT/EP2009/004655
(87) International publication number: WO 2009/156180

(56) References cited:
- WO-A1-2008/130641
- JP-A- H04 128 207
- US-A- 5 164 477
- LUXENHOFER R ET AL: "Click Chemistry with Poly(2-oxazoline)s", MACROMOLECULES, AMERICAN CHEMICAL SOCIETY, US, vol. 39, no. 10, 25 April 2006 (2006-04-25), pages 3509-3516, XP002464187, ISSN: 0024-9297, DOI: DOI:10.1021/MA052515M cited in the application
- STEPHAN HUBER ET AL: "Modulation of the lower critical solution temperature of 2-Alkyl-2-oxazoline copolymers", COLLOID AND POLYMER SCIENCE ; KOLLOID-ZEITSCHRIFT UND ZEITSCHRIFT FÜR POLYMERE, SPRINGER, BERLIN, DE, vol. 286, no. 4, 14 November 2007 (2007-11-14), pages 395-402, XP019628549, ISSN: 1435-1536
- CHEON LEE S ET AL: "Polymeric micelles of poly(2-ethyl-2-oxazoline)-block-poly(@?-ca prola ctone) copolymer as a carrier for paclitaxel", JOURNAL OF CONTROLLED RELEASE, ELSEVIER, AMSTERDAM, NL, vol. 89, no. 3, 20 May 2003 (2003-05-20), pages 437-446, XP004423305, ISSN: 0168-3659, DOI: DOI:10.1016/S0168-3659(03)00162-7

## Description

The present invention relates to pharmaceutical or diagnostic compositions and formulations which comprise one or more drugs as hydrophobic active compounds, together with a delivery system which assists in the solubilisation of these compounds.

Many of the most potent drugs and drug candidates are not water soluble. Formulation of poorly soluble drugs, such as paclitaxel (PTX) with a water solubility of approx. 1µg/mL, remains a major challenge in drug delivery (Huh, K. M., et al., J. Controlled Release 126, 122-129 (2008); Dabholkar, R. D., et al. Int. J. Pharm. 315, 148-157 (2006); Yang, T., et al., Int. J. Pharm. 338, 317-326 (2007); Torchilin, V.P., Cell. Mol. Life. Sci 61, 2549-2559 (2004); Haag, R., Angew. Chem. Int. Ed. 43, 278-282 (2004)). The current clinical formulation of PTX, Taxol®, contains less than 1% w/w of active drug but 99% w/w of excipients know to cause considerable side effects for the patients. Similar problems are encountered with active agents in other technical areas, such as plant protection, etc. Various methods to solubilize or disperse active agents have been developed. Traditional methods are typically based an the use of solvents, surfactants or chelating agents. These methods have one or more disadvantages related to toxicity of the excipients, limited stability of the formulations in aqueous media, in particular upon dilution, or difficult formulation procedures.

More recently, liposomes (Wu, J., et al., Int. J. Pharm. 316, 148-153 (2006)), micro- and nanoparticles (Desai, N.P. et al., Anti-Cancer Drugs 19, 899-909 (2008)) and polymer micelles (Huh, K. M., et al., J. Controlled Release 126, 122-129 (2008); Konno, T., et al., J. Biomed. Mat. Res., Part A, 65A, 210-215 (2002); Kim, S.C., et al., J. Controlled Release 72, 191-202 (2001)) have been studied intensively as solubilisation/drug delivery systems, each approach having advantages and disadvantages. One major limitation of polymer micelles is the loading capacity and the total amount of drug that can be solubilized. U.S. Pat. App. 20040185101 discloses polymeric compositions with the capability to solubilize hydrophobic drugs in aqueous media. However, the loading capacity of these compositions is limited with a loading capacity of e.g. < 10% (w/w) for paclitaxel, or less than 1% (w/w) for cyclosporin A.

Poly(2-oxazoline)s have recently attracted considerable attention for biomedical applications. Of particular interest are hydrophilic poly(2-methyl-2-oxazoline) (PMeOx) and poly(2-ethyl-2-oxazoline) (PEtOx) as they exhibit stealth (Zalipsky, S., et al., J. Pharm. Sci. 85, 133-137 (1996); Woodle, M. C., et al., Bioconjugate Chem. 5, 494-496 (1994)) and protein repellent effects (Konradi, R., et al., Langmuir 24, 613-616 (2008)) and undergo rapid renal clearance (Gaertner, F.C., et al., J. Controlled Release 119, 219-300 (2007)) similar to poly(ethylene glycol), a commonly used polymer for injectable drug delivery systems.

JP H04 128207 A discloses a cosmetic material that contains a copolymer composed of two or more specific types of 2-oxazoline monomers.

It is the aim of the present invention to provide compositions containing a delivery system which allows active agents, in particular hydrophobic active agents, to be efficiently solubilised and/or formulated. In particular, the compositions should be simple to prepare and provide a high loading capacity for the subject active agent.

In order to solve this problem, the present invention provides a pharmaceutical or diagnostic composition, comprising:
(a) at least one block copolymer comprising at least one poly(2-oxazoline) block A consisting of repeating units of formula (I) 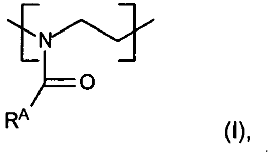
   wherein R^{A} is a hydrocarbon group, optionally substituted with -OH, -SH, -COOH, -NR'₂, -COOR', -CONR', -CHO, with R' representing H or C₁₋₃ alkyl, and with R^{A} being selected such that the repeating unit of formula (I) is hydrophilic;
   and at least one poly(2-oxazoline) block B consisting of repeating units of formula (II) 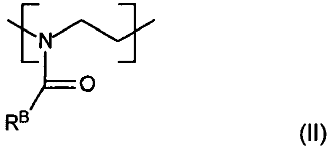 wherein R^{B} is a hydrocarbon group optionally substituted with halogen, -OH, -SH, -COOH, -NR"₂, -COOR", -CONR", -CHO, with R" representing H, alkyl or alkenyl, and with R^{B} being selected such that the repeating unit of formula (II) is more hydrophobic than the repeating unit of formula (I); and
(b) one or more drugs as hydrophobic active agent(s).

**Fig. 1** shows the fluorescence intensity and I₁/I₃ ratios of pyrene solutions in dependence of concentration of certain copolymers used in the context of the invention at 25 °C.
**Fig. 2** shows the results of the solubilization of PTX with amphiphilic copolymers using the film method.
**Fig. 3** shows the results of dynamic light scattering measurements on drug loaded micelles -formed by the compositions according to the invention.
**Fig. 4** shows the results of the assays for cytotoxicity of polymers used in the context of the invention in various cell lines.
**Fig. 5** shows the paclitaxel dose dependent viability of multi-drug resistant MCF7/ADR cells for compositions according to the invention.
**Fig. 6** shows as relative tumor weights (A) and tumor inhibition (B) in mice comparing negative controls, treatment with compositions according to the invention and a commercial product.

As noted above, in the units of formula (I), R^{A} is a hydrocarbon group, optionally substituted with -OH, -SH, -COOH, -NR'₂, -COOR', -CONR', -CHO, with R' representing H or C₁₋₃ alkyl, and with R^{A} being selected such that the repeating unit of formula (I) is hydrophilic.

Preferably, R^{A} is selected from a C₁₋₈ hydrocarbon group, preferably a C₁₋₆ hydrocarbon group, more preferably a C₁₋₃ and in particular a C₁₋₂ hydrocarbon group, all of which may be optionally substituted. Preferred as hydrocarbon groups are alkyl groups.

As will be understood, the hydrophilic property of the unit of formula (I) as defined above will depend on the size of the hydrocarbon group in R^{A}. If a small hydrocarbon group is selected, such as methyl or ethyl, the resulting group R^{A}, unsubstituted or substituted with the above substituents, will always be hydrophilic. If a larger hydrocarbon group is selected, the presence of substituents may be advantageous to introduce additional polarity to the unit of formula (I). Thus, preferably, R^{A} is selected, independently for each occurrence, from methyl and ethyl optionally substituted with halogen, -OH, -SH, -COOH, -NR'₂, -COOR', -CONR', - CHO, with R' representing H or C₁₋₃ alkyl, and particularly preferably R^{A} is selected from methyl or ethyl.

In the units of formula (II), R^{B} is a hydrocarbon group optionally substituted with halogen, -OH, -SH, -COOH, -NR"₂, -COOR", -CONR", -CHO, with R" representing H, alkyl or alkenyl, and with R^{B} selected such that the repeating unit of formula (II) is more hydrophobic than the repeating unit of formula (I). If R" is alkyl or aryl, preferred are C₁₋₈ alkyl or aryl groups. Halogen substituents, if present, are preferably selected from Cl and F.

Preferably, R^{B} is selected from a C₃₋₂₀ hydrocarbon group, preferably a C₃₋₁₂ hydrocarbon group, more preferably a C₃₋₆ and in particular a C₄₋₆ hydrocarbon group, all of which may be optionally substituted. However, it is further preferred that the hydrocarbon group does not carry a substituent.

Preferred as hydrocarbon groups are aliphatic or aromatic groups, such as alkyl groups, aryl groups or alkaryl groups. More preferred are alkyl groups, such as propyl, butyl, pentyl, hexyl, heptyl, octyl or nonyl, even more preferred are C₄₋₆ alkyl groups, i.e. butyl, pentyl, hexyl and specifically preferred are butyl groups, particularly n-butyl.

The terms "hydrophilic" and "hydrophobic" as used herein have a well recognized meaning in the art. "Hydrophilic" designates a preference of a substance or moiety for aqueous environments, i.e. a hydrophilic substance or moiety is more readily dissolved in or wetted by water than by non-polar solvents, such as hydrocarbons. "Hydrophobic" designates a preference for apolar environments i.e. a hydrophobic substance or moiety is more readily dissolved in or wetted by non-polar solvents, such as hydrocarbons, than by water. The term "amphiphilic" denotes the simultaneous presence of hydrophilic and less hydrophilic or more hydrophobic moieties in a compound, as frequently encountered in surfactants. To that extent, the copolymers used in the context of the invention are also referred to herein as amphiphilic copolymers since they comprise hydrophilic moieties and moieties which are less hydrophilic /more hydrophobic, respectively.

If necessary, i.e. if it is not readily apparent from the chemical structure of R^{A} and R^{B} that a specific unit of formula (II) is more hydrophobic than a given unit of formula (I), this can be verified, e.g., by preparing comparable homopolymers of the respective units and determining their logP value under the same conditions. The logP value, as commonly known, is the logarithm of the partition coefficient observed for a species A between water and n-octanol. In particular, the partition coefficient P of a species A is defined as the ratio P = [A]_{n-octanol} / [A]_{water}, wherein [A] indicates the concentration of A in the respective phase. The more hydrophilic substance will have higher concentrations in water. Typically, the volumes of water and octanol are the same for the measurement.

In a preferred embodiment, the correct selection of R^{B} to provide units of formula (II) which are more hydrophobic than those of formula (I) can be verified by determining the critical micelle concentration (CMC) of a copolymer containing these units according to the procedure disclosed in detail below. If a CMC can be observed, the requirement regarding the hydrophilic nature of the units of formula (I) and the more hydrophobic/less hydrophilic nature of the units of formula (II) is reliably fulfilled.

The requirement that the units of formula (I) are hydrophilic and the units of formula (II) are more hydrophobic than those of formula (I) will also be reliably fulfilled for any possible combination of the following preferred embodiments of R^{A} and R^{B}, as will be apparent from the structures thereof. Namely, R^{A} is preferably selected from methyl and ethyl optionally substituted with -OH, -SH, -COOH, -NR'₂, -COOR', -CONR', -CHO, with R' representing H or C₁₋₃ alkyl, and particularly preferably R^{A} is selected from methyl or ethyl; and R^{B} is selected from an unsubstituted C₃₋₂₀ hydrocarbon group, preferably a C₃₋₁₂ hydrocarbon group, more preferably a C₃₋₆ and in particular a C₄₋₆ hydrocarbon group, wherein preferred hydrocarbon groups are aliphatic or aromatic groups, such as alkyl groups, aryl groups or alkaryl groups. More preferred are alkyl groups, such as propyl, butyl, pentyl, hexyl, heptyl, octyl or nonyl, even more preferred are C₄₋₆ alkyl groups, i.e. butyl, pentyl, hexyl and specifically preferred is a butyl, particularly n-butyl.

It should be understood that reference to aqueous environments, aqueous media, aqueous solutions or the like in this application, means solvent systems wherein 50 % (v/v) or more, preferably 70 % or more, more preferably 90 % or more and in particular substantially 100 % of the total volume of solvent(s) is water.

A copolymer comprising repeating units of formula (I) and (II) above can be conveniently prepared via ring opening polymerization of 2-substituted 2-oxazolines (or 2-substituted 4,5-dihydro oxazoles according to IUPAC nomenclature). Therefore, the polymers used in the context of the invention are also referred to as poly(2-oxazoline)s.

The copolymer according to the invention can comprise other repeating units in addition to repeating units (I) and (II) above. However, it is preferred that the major portion of all repeating units, i.e. more than 50 %, more preferably more than 75 %, further preferably more than 90 % and particularly preferably 100 %, based on the total number of repeating units, are repeating units of formula (I) or (II) as defined above. It will be understood that all repeating unit of formula (II) contained in the copolymer will be more hydrophobic than any of the repeating units of formula (I) contained in the copolymer.

The ratio of repeating units (I) to repeating units (II), in terms of the numbers of repeating units, typically ranges from 20:1 to 1:2, preferably from 10:1 to 1:1, and more preferably from 7:1 to 3:1.

With respect to the arrangement of the repeating units (I) and (II) above, the copolymers according to the invention are block copolymers.

The term "block copolymer" is used herein in accordance with its established meaning in the art to refer to copolymers wherein repeating units of a defined type are organized in blocks, i.e. repeating units of the same type are polymerized sequentially adjacent to each other as opposed to, for example, sequences of randomly alternating repeating units of different types.
In other words, the blocks of a block copolymer, such as blocks A and B to be further discussed below, represent polymeric entities themselves, obtained by the polymerization of monomers which are identical or which have certain common characteristics.
The block copolymer to be used in accordance with the present invention comprises at least one poly(2-oxazoline) block A consisting of repeating units of formula (I) as defined above (which, due to the presence of the respective units, is hydrophilic), and at least one poly(2-oxazoline) block B consisting of repeating units of formula (II) as defined above (which, due to the presence of the respective units, is more hydrophobic than block A).
The block copolymer according to the invention can be referred to as an amphiphilic copolymer due to the presence of at least one hydrophilic moiety (block A) and at least one less hydrophilic or more hydrophobic moiety (block B).

Preferably at least one block A of the block copolymer, more preferably all blocks A in the case of multiple occurrences, is (are) represented by formula (Ia): 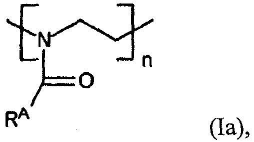 wherein R^{A} represents a methyl or ethyl group, preferably a methyl group, and n indicates the number of repeating units within the block A. It represents preferably an integer of 5 or more, more preferably 10 or more, and particularly 20 or more. It is generally below 300, preferably 200 or less, more preferably 100 or less and in particular 50 or less.

Preferably, at least one block B of the block copolymer, more preferably all blocks B in the case of multiple occurrences, is (are) represented by formula (Ila): 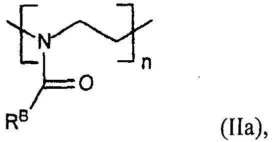 wherein R^{B} is a C₃₋₂₀ hydrocarbon group, preferably a C₃₋₁₂ hydrocarbon group, more preferably a C₃₋₆ and in particular a C₄₋₆ hydrocarbon group. Preferred as hydrocarbon groups are aliphatic or aromatic groups, such as alkyl groups, aryl groups or alkaryl groups. More preferred are alkyl groups such as propyl, butyl, pentyl, hexyl, heptyl, octyl or nonyl, even more C₄₋₆ alkyl groups, i.e. butyl, pentyl, hexyl and specifically preferred are butyl groups, particularly n-butyl. The variable n preferably represents an integer of 5 or more, more preferably of 10 or more. It is generally below 300, preferably 200 or less or 100 or less, and more preferably 50 or less.

The block copolymer used as a drug delivery system in the context of the invention contains at least one block A and at least one block B as defined above. It may contain one or more additional blocks which are different from A or B. However, it is preferred that the block copolymer contains exclusively blocks falling under the definitions and preferred definitions of A and B above. More preferably, all repeating units of the block copolymer are repeating units of formula (I) or (II) above.

As for the arrangement of blocks A and B in the copolymer used in the context of the invention, preferred structures of the copolymer can be indicated as (AB)ₘ or (BA)ₘ with m being 1, 2 or 3, as ABA, or as BAB. It is more preferred that the block copolymer is an AB or BA diblock copolymer or an ABA triblock copolymer.

Thus, in a particularly preferred embodiment of the invention, the polymeric entities of the block copolymer consist of (an) A block(s) consisting of polymerized 2-methyl-2-oxazoline or 2-ethyl-2-oxazoline (also referred to herein as "poly(2-methyl-2-oxazoline) block" or "poly(2-ethyl-2-oxazoline) block") and (a) B block(s) consisting of polymerized 2-(C₄₋₆ alkyl)-2-oxazoline. Even more preferred is such copolymer with (an) A block(s) consisting of polymerized 2-methyl-2-oxazoline or 2-ethyl-2-oxazoline and (a) B block(s) consisting of polymerized 2-butyl-2-oxazoline (also referred to as "poly(2-butyl-2-oxazoline) block"). Further preferred is an AB or ABA di- or triblock-copolymer of the above constitution.

The copolymers used in the context of the invention preferably exhibit a number average molecular mass (Mₙ), as determined by gel permeation chromatography, ranging from 3 to 30, more preferably from 4 to 25, and particularly from 6 to 20 kg/mol. Polydispersity indices (PDI = M_{w}/Mₙ with M_{w} being the weight average molecular mass) are typically below 1.3, preferably below 1.25, and can be as low as 1.001.

It will be understood that compositions comprising combinations, e.g. mixtures or blends of two or more different copolymers are also encompassed by the invention, e.g. combinations of copolymers containing different groups R^{A} and or R^{B}, or combinations of copolymers showing different arrangements of their repeating units, e.g. combinations of a random polymer and a block copolymer.

The copolymers used in the context of the invention can be prepared by polymerization methods known in the art. For example, poly(2-oxazoline)s can be prepared by living cationic ring opening polymerization. The preparation of random copolymers, gradient copolymers and block copolymers, is described in detail, e.g., by R. Luxenhofer and R. Jordan, Macromolecules 39, 3509 - 3516 (2006), T. Bonné et al., Colloid. Polym. Sci., 282, 833-843 (2004) or T. Bonne et al. Macromol. Chem. Phys. 2008, 1402-1408, (2007).

As regards the active agent (or active compound) contained in the compositions according to the invention, it will be understood that the compositions are used for the formulation of active agents which are hydrophobic active agents, or which may be non-water soluble active agents. Thus, it is preferred that active agents are comprised which have a solubility in water, e.g. ion-exchanged water, at 20 °C, of less than 1 mg/mL, preferably less than 0.1 mg/mL or even less than 0.01 mg/mL, and in particular with a solubility of less than 0.001 mg/mL. Preferably, this limited solubility is shown in water over the pH range of 4 to 10.

The active agent (or active compound) for use in the context of the present invention is a bioactive agent (or bioactive compound) for use in therapy (i.e. a drug) or in diagnosis.

Specific examples of the bioactive agent include, but are not limited to, drugs in the following categories: drugs acting at synaptic and neuroeffector junctional sites, drugs acting an the central nervous system, drugs that influence inflammatory responses, drugs that affect the composition of body fluids, drugs affecting renal function and electrolyte metabolism, cardiovascular drugs, drugs affecting gastrointestinal function, drugs affecting uterine motility, chemotherapeutic agents for hyperproliferative diseases, particularly cancer, chemotherapeutic agents for parasitic infections, chemotherapeutic agents for microbial diseases, antineoplastic agents, immunosuppressive agents, drugs affecting die blood and blood-forming organs, hormones and hormone antagonists, dermatological agents, heavy metal antagonists, vitamins and nutrients, vaccines, oligonucleotides and gene therapies. Specific drugs which may be mentioned as being suitable for use in the present invention include amphotericin B, nifedipine, griseofulvin, taxanes including paclitaxel and docetaxel, doxorubicin, daunomycin, indomethacin, ibuprofen, etoposide, cyclosporin A, vitamine E, and testosterone, in particular paclitaxel, cyclosporine A and amphotericin B. As noted above, particular utility is shown by the invention for active agents considered to be non water-soluble.

It will be understood that compositions comprising combinations, e.g. mixtures or blends of two or more active agents, such as two drugs, are also encompassed by the invention.

It is generally preferred that the copolymer forms aggregates in the compositions according to the invention, and it is further preferred that the aggregates are formed such that the copolymer aggregates incorporate the active agent. A particularly preferred form of such an aggregate is a micelle. A micelle, as referred to herein, is generally an aggregate of amphiphilic copolymers presenting a hydrophilic corona formed by the hydrophilic parts of the copolymer and sequestering the hydrophobic parts of said amphiphilic copolymers in the interior of the micelle. Particularly suitable copolymers for the formation of micelles are the block copolymers discussed above as a preferred embodiment of the copolymers. Micelles according to the invention are three-dimensional entities. Generally, micelles are formed when the concentration of the constituent amphiphilic molecules in an aqueous solution, exceeds a certain value. This, value is referred to as critical micelle concentration (CMC) which may be determined by using a fluorescent probe, such as pyrene, which partitions into the hydrophobic core of the micelles formed above the CMC value. More specifically, micelles according to the invention form, for example, by self-aggregation of the amphiphilic block copolymers in hydrophilic, preferably aqueous solutions. Upon formation of the micelles, the hydrophilic regions of said amphiphilic copolymers are in contact with the surrounding solvent, whereas the hydrophobic regions are facing towards the centre of the micelle. In the context of the invention, the centre of a micelle typically incorporates the hydrophobic active agent. A micelle may also be referred to as a "polymeric nanoparticle" because of its size in the nanometer range and its constituents being of polymeric nature.

The copolymers, especially the block copolymers used in the context of the invention typically have low CMCs which are smaller than 250 mg/L. The CMCs generally range from 5 to 150 or even 5 to 100 mg/L.

Aggregates, particularly micelles of variable size may be formed by the pharmaceutical compositions according to the invention, depending on factors such as the molecular weight of the copolymer used, or the drug load. Generally preferred are aggregates or micelles within a size range of 5 - 500 nm. However, it is possible to advantageously form aggregates or micelles with sizes ranging from 5 to 100 or 10 to 50 nm or even from 10 to 30 nm, as determined by dynamic light scattering, which are particularly suitable for intravenous administration. Advantageously, the micelles typically have narrow particle size distributions (PDI ≤ 0.2 or even ≤ 0.1).

Typically, the aggregates, particularly micelles, form in water or aqueous media. Thus, the aggregates, particularly micelles, of a composition according to the invention, may be formed, e.g., by the thin film dissolution method. In this method, the copolymer and the active agent are dissolved in a common solvent, such as acetonitrile or dimethylsulfoxide. After removal of the solvent (e.g. by a stream of inert gas, gentle heating and/or application of reduced pressure), films formed by the polymer and the active agent can be easily dissolved in water or aqueous solutions and may be tempered at increased temperatures. When the films are dissolved, the aggregates, preferably micelles, form. The stability of the aggregates allows the resulting solutions to be dried to form a powder. For example, they can freeze-dried, generally without the need for a cryoprotectant, and reconstituted in water or aqueous solutions without compromising loading capacities or particle integrity.

As a result of the use of the copolymers described above, the compositions according to the invention typically form aggregates soluble in water or aqueous solutions where they are stable at least 12 h at room temperature and at elevated temperatures, especially at temperatures below 40°C, that allow for the parenteral administration of said compositions in animal in general and human in particular.

The weight ratio of active agent(s) to copolymer(s) in the compositions according to the invention is typically 1:20 or higher, such as 1:10 or higher. Preferably, the weight ratio is at least 1:9, more preferably at least 2:8, even more preferably at least 3:7 and most preferably 4:6. It is generally 1:1 or less, if necessary 8:10 or less.

The compositions according to the invention can advantageously achieve an active load or drug load (i.e. a ratio of the weight of the active agent or drug to the sum of the weights of the active agent and the block copolymer, given as percentage) of 10 % or more, preferably 25 % or more, more preferably 30 % or more, even more preferably 35 % or more and in particular 40 % or more. It was particularly surprising that a sufficient water solubility could be eventually be achieved for compositions according to the invention having such high drug loads even with active agents with a solubility of less than 10 µg/mL or even less than 5 µg/mL, such as paclitaxel. Thus, for example, the compositions according to the invention allow for a solubilisation of paclitaxel of more than 7 mg/mL of paclitaxel, particularly 8 mg/mL or more, in water and aqueous solutions.

The high capacity even for hydrophobic active agents coincides with unusual values obtained from pyrene fluorescence spectroscopy of the pharmaceutical compositions according to the invention. The ratio of I₁ and I₃ bands in the fluorescence emission spectrum of pyrene is a measure of the polarity (K. Kalyanasundaram, J.K. Thomas, J. Am. Chem. Soc. 1977, 99, 2039-2044) of the environment of the pyrene probe. In an aqueous or similarly polar environment this ratio is typically found between 1.6 and 1.9 (K.W.Street, Jr., W.W.Acree, Jr. Analyst 1986, 111, 1197-1201). In the presence of polymer micelles, a less polar environment is available for pyrene and the I₁/I₃ ratio usually decreases concomitantly with an increasing overall fluorescence intensity. Quite surprisingly, with the copolymers and especially the block copolymers described herein, the opposite can be observed as the I₁/I₃ ratio increases to values above 2.0, preferably above 2.1 or even above 2.2, e.g. up to 2.35.

Due to the high solubilising efficiency observed for the copolymers and particularly the block copolymers described above, it is generally sufficient for compositions, in particular pharmaceutical compositions in accordance with the invention in the form of aqueous solutions if the content of the copolymer ranges from concentrations as low as 1 mg/mL, preferably 2 mg/mL, to concentrations of 100 mg/mL, preferably to 50 mg/mL or 20 mg/mL. Since the copolymers are biocompatible, i.e. non-toxic, and undergo rapid renal clearance, high concentrations are not critical, but are generally not required. Compared to formulations of hydrophobic drugs currently on the market, this allows a significant reduction of the amount of solubilizer subjects recieve upon parenteral administration of the drug, thus reducing the risk of adverse health effects.

As a matter of fact, the block copolymer described herein can reduce the amount of excipient needed to solubilize the same amount of paclitaxel by approx. 100 and 9 times, as compared to Cremophor EL/ethanol (CrEL) and Abraxane™, respectively.

Furthermore, the loading efficiency (i.e. (amount of solubilised active agent/amount of initially charged active agent) *100%) can be 100% and is generally very high (> 80%) for the compositions according to the invention. This is a significant advantage as high loading efficiencies are of importance for commercial applications for the reduction of production costs.

As explained above, the copolymers according to the invention can be used to increase the solubility of active agents which are hydrophobic active agents or which may be non-water soluble active agents, in water or aqueous solutions, i.e. they can act as a solubilizer for these compounds.

As a result, in one preferred embodiment, the compositions according to the invention further contain water to form an aqueous solution, emulsion or suspension, and particularly preferably they are aqueous solutions of the active agent and the copolymer. It will be understood that the term "solution" comprises, in this specific context, colloidal solutions as they may be formed by micelles in water. However, since the copolymers used in the context of the invention allow the compositions to be lyophilized without compromising the activity and the stability of the active agent and without the need for a cryoprotectant, powders, especially lyophilized powders, form another preferred embodiment of the compositions according to the invention. These powders may be conveniently reconsitituted in water or aqueous solutions.

Thus, the poly(2-oxazoline) based copolymers described herein can serve, e.g., as a versatile high capacity drug delivery system even for hydrophobic and structurally diverse drugs such as paclitaxel, cyclosporin A and amphotericin B.

Other embodiments of the present invention are summarized in the following items
1. Pharmaceutical composition comprising
   (a) at least one biocompatible water soluble amphiphilic block copolymer comprising of at least one block A and at least one block B, wherein A is a hydrophilic polymer selected from hydrophilic poly(2-oxazoline)s as defined herein and B is selected from amphiphilic or hydrophobic poly(2-oxazoline)s as defined herein and
   (b) a hydrophobic bioactive compound,
   that form soluble aggregates in water or aqueous solutions that are stable at least 12 h at room temperature and at elevated temperatures, especially at temperatures below 40°C, that allow for the parenteral administration of said compositions in animal in general and human in particular.
2. Pharmaceutical compositions of item 1, wherein B is represented by the following structure of formula (III): 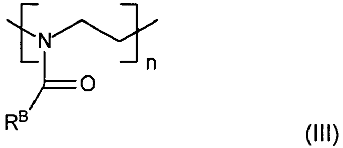 wherein R^{B} is a hydrophobic side chain (comprising a saturated aliphatic chain, an unsaturated aliphatic chain, a saturated aliphatic ring or an unsaturated aliphatic ring or mixtures thereof) and n is selected between 1 and 300.
3. Pharmaceutical composition according to item 1 or 2, wherein hydrophobic bioactive compound comprises peptides, peptoides, polyenes, macrocyles, glycosides, terpenes, terpenoids, aliphatic and aromatic compounds and their derivatives and other compounds of a solubility in water or aqueous media at a pH range of 4 - 10 lower than 1 mg/mL, preferably lower 100 µg/mL, even more preferably lower than 50 µg/mL. and most preferably lower than 10 µg/mL.
4. Pharmaceutical compositions according to any of items 1 to 3, wherein the hydrophobic bioactive compound is selected from amphotericin B, nifedipine, griseofolvin, paclitaxel, docorubicin, daunomycin, indomethacain, ibuprofen, etoposide and cyclosporine A.
5. Pharmaceutical compositions according to any of items 1 to 3, wherein the hydrophobic bioactive compound is paclitaxel.
6. Pharmaceutical compositions according to any of items 1 to 5, wherein the AB block copolymers are attached through stable or labile linkages to form compounds that can be depicted as (AB)ₘ, with m ranging from 2-100, forming, for example, linear or star-like block copolymers, graft block copolymers, dendrimer based or hyperbranched blockcopolymers.
7. Pharmaceutical compositions according to any of items 2 to 6, wherein the hydrophobic side chain R comprises 3-6 carbon atoms.
8. Pharmaceutical compositions according to any of items 1 to 7, wherein the amphiphilic block copolymer comprises a block that consists in part or completely of repeating units derived from 2-butyl-2-oxazoline
9. Pharmaceutical compositions according to any of items 1 to 8, wherein the hydrophilic polyoxazoline is selected from poly(2-methyl-2-oxazoline) or poly(2-ethyl-2- oxazoline).
10. Pharmaceutical compositions according to any of items 1 to 9, wherein the soluble aggregates in aqueous media are in the size range of 5 - 200 nm, preferably 10-100 nm.
11. Pharmaceutical compositions according to any of items 1 to 10, comprising the hydrophobic bioactive compound and amphiphilic block copolymer in a weight ratio of at least 1:9, preferable 2:8, more preferable 3:7 and most preferable 4:6.

Due to the inherent versatility of the pharmaceutical compositions forming a preferred embodiment according to the invention as regards bioactive agents/compounds to be incorporated, it will be understood that the compositions are suitable for the treatment or prevention of a wide variety of diseases or disorders such as cancer, neurodegenerative diseases, hepato-biliary diseases, cardiovascular diseases or pulmonary diseases. The invention also encompasses the use of the block copolymers as defined above for the preparation of a pharmaceutical composition for the treatment or prevention of any of these diseases. Moreover, diagnostic applications of the compositions according to the inventions are also envisaged.

The term "cancer", in accordance with the present invention refers to a class of diseases or disorders characterized by uncontrolled division of cells and the ability of these to spread, either by direct growth into adjacent tissue through invasion, or by implantation into distant sites by metastasis (where cancer cells are transported through the bloodstream or lymphatic system).

The term "neurodegenerative disease", in accordance with the present invention refers to a class of diseases or disorders wherein neurons deteriorate and due to the inability of the body to regenerate neurons (except a small number neural stem cells) the cells for example of the brain or spinal chord cannot be adequately regenerated. Symptoms encompass ataxia as well as dementia in affected individuals.

The term "gastrointestinal and hepato-biliary disease", in accordance with the present invention relates to diseases or disorders affecting the liver, gall bladder and bile ducts. Such diseases and disorders include, for example, cirrhosis, hepatitis, virally induced hepatitis, liver tumors, fatty liver, polycystic liver, Morbus Crohn, Colitis ulcerosa and cholangiocarcinoma.

The term "cardiovascular disease", in accordance with the present invention relates to a class of diseases or disorders involving the heart an/or blood vessels.

The term "pulmonary diseases", as used in accordance with the present invention relates to diseases affecting the respiratory system and can be classified into obstructive, i.e. impeding the rate of low into and out of the lungs, and restrictive, i.e. reduction in the functional volume of the lungs, conditions. Such diseases include, for example, asthma, bronchitis, asbestosis, fibrosis, sarcoidosis, lung cancer, pneumonia, pulmonary edema and pulmonary hypertension.

The pharmaceutical compositions according to the invention may optionally be formulated together with one or more further pharmaceutically acceptable excipients, such as carriers, diluents, fillers, disintegrants, lubricating agents, binders, colorants, pigments, stabilizers, preservatives, and/or antioxidants.

The pharmaceutical compositions can be formulated by techniques known to the person skilled in the art, such as the techniques published in Remington's Pharmaceutical Sciences, 20th Edition. The pharmaceutical compositions can be formulated as dosage forms for oral, parenteral, such as intramuscular, intravenous, subcutaneous, intradermal, intraarterial, rectal, topical, pulmonary or vaginal administration. Dosage forms for oral administration include coated and uncoated tablets, soft gelatin capsules, hard gelatin capsules, lozenges, troches, solutions, emulsions, suspensions, syrups, elixirs, powders and granules for reconstitution, dispersible powders and granules, medicated gums, chewing tablets and effervescent tablets. Dosage forms for parenteral administration include solutions, emulsions, suspensions, dispersions and powders and granules for reconstitution. Emulsions are a preferred dosage form for parenteral administration. Dosage forms for rectal and vaginal administration include suppositories and ovula. Dosage forms for pulmonary administration/pulmonary delivery can be administered via inhalation and insufflation, for example by a metered dose inhaler. Dosage forms for topical administration include creams, gels, ointments, salves, patches and transdermal delivery systems.

The pharmaceutical compositions according to the invention may be administered to a subject by any convenient route of administration, whether systemically/peripherally or at the site of desired action, including but not limited to one or more of: oral (e.g. as a tablet, capsule, or as an ingestible solution), topical (e.g., transdermal, intranasal, ocular, buccal, and sublingual), parenteral (e. g., using injection techniques or infusion techniques, and including, for example, by injection, e.g. subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, or intrasternal by, e.g., implant of a depot, for example, subcutaneously or intramuscularly), pulmonary (e.g., by inhalation or insufflation therapy using, e.g., an aerosol, e.g. through mouth or nose), gastrointestinal, intrauterine, intraocular, subcutaneous, ophthalmic (including intravitreal or intracameral), rectal, and vaginal. Oral and parenteral, especially intravenous administration is generally preferred since the compositions according to the invention provide a sufficient solubility and bioavailability for these routes even when hydrophobic active agents are used.

If the pharmaceutical compositions are administered parenterally, then examples of such administration include one or more of: intravenously, intraarterially, intraperitoneally, intrathecally, intraventricularly, intraurethrally, intrasternally, intracranially, intramuscularly or subcutaneously administering the compounds pharmaceutical compositions, and/or by using infusion techniques. For parenteral administration, the compounds are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

The pharmaceutical compositions can also be administered orally in the form of tablets, capsules, ovules, elixirs, solutions or suspensions, which may contain flavoring or coloring agents, for immediate-, delayed-, modified-, sustained-, pulsed- or controlled-release applications.

The tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycolate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included. Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the agent may be combined with various sweetening or flavoring agents, coloring matter or dyes, with emulsifying and/or suspending agents and with diluents such as water, ethanol, propylene glycol and glycerin, and combinations thereof.

Alternatively, the pharmaceutical compositions can be administered in the form of a suppository or pessary, or it may be applied topically in the form of a gel, hydrogel, lotion, solution, cream, ointment or dusting powder. The compositions of the present invention may also be dermally or transdermally administered, for example, by the use of a skin patch.

The pharmaceutical compositions may also be administered by the pulmonary route, rectal routes, or the ocular route. For ophthalmic use, they can be formulated as micronized suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

For topical application to the skin, pharmaceutical compositions can be formulated as a suitable ointment containing the active compound suspended or dissolved in, for example, a mixture with one or more of the following: mineral oil, liquid petrolatum, white petrolatum, propylene glycol, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, 2-octyldodecanol, benzyl alcohol and water.

In view of the advantageous solubilising effects provided by the compositions according to the invention, it will be understood that they are preferably administered in forms and/or according to modes of administration which require solubility of the bioactive ingredient in water.

Typically, a physician will determine the actual dosage of the pharmaceutical compositions which will be most suitable for an individual subject. The specific dose level and frequency of dosage for any particular individual subject may be varied and will depend upon a variety of factors including the disorder or disease to be treated or prevented, the specific bioactive compound employed, the metabolic stability and length of action of that compound, the age, body weight, general health, sex, diet, mode and time of administration, rate of excretion, drug combination, the severity of the particular condition, and the individual subject undergoing therapy.

A proposed, yet non-limiting dose of the compositions according to the invention for administration to a human (of approximately 70 kg body weight) may be 0.1 µg to 10 g, preferably 0.1 mg to 0.5 g, based on the weight of the active agent (i.e. the drug) per unit dose. The unit dose may be administered, for example, 1 to 4 times per day. The dose will depend on the route of administration. It will be appreciated that it may be necessary to make routine variations to the dosage depending on the age and weight of the patient/subject as well as the severity of the condition to be treated. The precise dose and route of administration will ultimately be at the discretion of the attendant physician or veterinarian.

The subject or patient, such as the subject in need of treatment or prevention, to which the compositions according to the invention are administered, is generally a mammal. In the context of this invention, it is particularly envisaged that mammals are to be treated, besides humans, which are economically or agronomically important. Non-limiting examples of agronomically important animals are sheep, cattle and pig, while, for example, cats and dogs may be considered as economically important animals. Preferably, the subject/patient is a human.

The term "treatment of a disorder or disease" as used herein is well known in the art. "Treatment of a disorder or disease" implies that a disorder or disease has been diagnosed in a patient/subject. A patient/subject suspected of suffering from a disorder or disease typically shows specific clinical and/or pathological symptoms which a skilled person can easily attribute to a specific pathological condition (i.e. diagnose a disorder or disease).

"Treatment of a disorder or disease" may, for example, lead to a halt in the progression of the disorder or disease (e.g. no deterioration of symptoms) or a delay in the progression of the disorder or disease (in case the halt in progression is of a transient nature only). "Treatment of a disorder or disease" may also lead to a partial response (e.g. amelioration of symptoms) or complete response (e.g. disappearance of symptoms) of the subject/patient suffering from the disorder or disease. "Amelioration" of a disorder or disease may, for example, lead to a halt in the progression of the disorder or disease or a delay in the progression of the disorder or disease. Such a partial or complete response may be followed by a relapse. It is to be understood that a subject/patient may experience a broad range of responses to a treatment (e.g. the exemplary responses as described herein above). Treatment of a disorder or disease may, inter alia, comprise curative treatment (preferably leading to a complete response and eventually to healing of the disorder or disease) and palliative treatment (including symptomatic relief).

Also the term "prevention of a disorder or disease" as used herein is well known in the art. For example, a patient/subject suspected of being prone to suffer from a disorder or disease as defined herein may, in particular, benefit from a prevention of the disorder or disease. Said subject/patient may have a susceptibility or predisposition for a disorder or disease, including but not limited to hereditary predisposition. Such a predisposition can be determined by standard assays, using, for example, genetic markers or phenotypic indicators. It is to be understood that a disorder or disease to be prevented in accordance with the present invention has not been diagnosed or cannot be diagnosed in said patient/subject (for example, said patient/subject does not show any clinical or pathological symptoms). Thus, the term "prevention" comprises the use of compounds of the present invention before any clinical and/or pathological symptoms are diagnosed or determined or can be diagnosed or determined by the attending physician.

### Examples

In the following examples, the invention will be illustrated in further detail. It will be understood that the embodiments disclosed herein are not to be construed as a limitation for the scope of the invention.

### 1. General Materials and Methods

All substances for the preparation of the polymers were purchased from Aldrich (Steinheim, Germany) and Acros (Geel, Belgium) and were used as received unless otherwise stated. 2-Butyl-2-oxazoline (BuOx) was prepare as recently described (Huber, S. and Jordan, R., Colloid Polym. Sci. 286, 395-402 (2008)). Methyl trifluoromethylsulfonate (MeOTf), 2-methyl-2-oxazoline (MeOx), 2-ethyl-2-oxazoline (EtOx), acetonitrile (ACN) and other solvents for polymer preparation were dried by refluxing over CaH₂ under dry nitrogen atmosphere and subsequent distillation prior to use. NMR spectra were recorded on a Bruker Avance III 400, Bruker ARX 300 or a Bruker AC 250 at room temperature. The spectra were calibrated using the solvent signals (CDCl₃ 7.26 ppm, D₂O 4.67 ppm). Gel permeation chromatography (GPC) was performed on a Waters system (pump mod. 510, RI-detector mod. 410, precolumn PLgel and two PL Resipore colunms (3 µm, 300 x 7,5 mm)) with N,N-dimethyl acetamide (DMAc) (75 mmol/L LiBr, 80 °C, 1 mL/min) as eluent and calibrated against PMMA standards. Dynamic light scattering was performed using a Zetasizer Nano-ZS (Malvern Instruments Inc., Southborough, MA) at room temperature.

### 2. Synthetic procedures

The polymerizations and work-up procedures were carried out according to the procedure described previously (Luxenhofer, R. and Jordan, R. , Macromolecules 39, 3509-3516 (2006); Bonné, T. B., et al., Colloid Polym. Sci. 282, 833-843 (2004))

Exemplary, the preparation of Methyl-P[MeOx₂₇-b-BuOx₁₂-b-MeOx₂₇]-piperidine **(P1)** was performed as follows (wherein "Methyl" indicates that the polymer carries a terminal methyl group, "P" indicates that the part within the brackets is the polymer part, "MeOx" stands for 2-methyl-2-oxazoline derived polymer units, the indices indicate the number of units in the respective polymer block, "b" indicates the start of a new block, "BuOx" stands for 2-butyl-2-oxazoline derived polymer units, and piperidine is the other terminal group of the polymer chain):
Under dry and inert conditions 32.2 mg (0.2 mmol, 1 eq) of methyl trifluoromethylsulfonate (methyl triflate, MeOTf) and 440 mg (5.17 mmol, 26 eq) of 2-methyl-2-oxazoline (MeOx) were dissolved in 3 mL dry acetonitrile at room temperature. The mixture was subjected to microwave irradiation (150 W maximum, 130 °C) for 15 min. After cooling to room temperature, the monomer for the second block, 2-butyl-2-oxazoline (256 mg, 2.01 mmol, 10 eq) were added and the mixture was irradiated the same way as for the first block. The procedure was repeated for the third block with 442 mg (5.19 mmol, 26 eq). Finally, P1 was terminated by addition of 0.1 mL piperidine (1.01 mmol, 5 eq) at room temperature. After stirring over night, an access of K₂CO₃ was added and the mixture was allowed to stir for several hours. The solvent was removed after filtration and 3 mL of chloroform were added to the residue. After precipitation from cold diethyl ether (approx. 10 times the amount of polymer solution) the product was obtained by centrifugation. The precipitation was performed in triplicate and the polymer was obtained as colorless powder (792 mg, 67 %, Mₜₕ= 5.8 kg/mol) after lyophilization from water. GPC (DMAc): Mₙ = 8.5 kg/mol (PDI 1.2₁); ¹H-NMR (CDCl₃, 298 K): δ = 3.45 (br, 255H, (N-C*H*₂C*H*₂)); 3.04/2.95 (m, 3H, N-C*H*₃^{Ini}); 2.43 -1.86 (m, 212H, CO-C*H*₃, CO-C*H*₂, C*H*₂^{Pid}); 1.56 (br, 29H, CH₂-C*H*₂-CH₂-); 1.32 (br, 28H, - C*H*₂-CH₃); 0.91 ppm (br, 37H, -C*H*₃^{butyl}), Mₙ = 6.2 kg/mol (MeOx₂₇-b-BuOx₁₂-b-MeOx₂₇).

### Preparation of Methyl-P[MeOx₃₇-b-BuOx₂₃-b-MeOx₃₇]-piperidine (P2)

P2 was obtained accordingly using 24 mg MeOTf (0.146 mmol, 1 eq), 333mg MeOx (3.91 mmol, 27 eq, 1^{st} block), 286 mg BuOx (2.25 mmol, 15 eq, 2^{nd} block) and 333 mg MeOx (3.91 mmol, 27 eq, 3^{rd} block) and 80 µL of piperidine as terminating reagent. The product was obtained as a colorless solid (795 mg, 83 %, Mₜₕ = 6.6 kg/mol).
GPC (DMAc): Mₙ = 10.4 kg/mol (PDI 1.1₈); ¹H-NMR (CDCl₃, 298 K): δ = 3.44 (br, 360H, (N-CH₂C*H*₂)); 3.03/2.94 (m, 3H, N-C*H*₃^{Ini}); 2.33 -1.9 (m, 279H, CO-C*H*₃, CO-C*H*₂, C*H*₂^{Pid}); 1.55 (br, 47H, CH₂-C*H*₂-CH₂-); 1.32 (br, 45H, -C*H*₂-CH₃); 0.91 ppm (br, 68H, -C*H*₃^{butyl}), Mₙ = 9.3 kg/mol (MeOx₃₇-b-BuOx₂₃-b-MeOx₃₇).

### Preparation of Methyl-P[MeOx₃₆-b-BuOx₃₀-b-MeOx₃₆]-piperidine (P3)

P3 was prepared accordingly using 24.7 mg methyltriflate (0.150 mmol, 1 eq) and 334 mg 2-methyl-2-oxazoline (3.9 mmol, 26 eq, 1st block). An aliquot of 136 mg (5% w/w) of the reaction mixture where removed for analysis of the first block with NMR and GPC. The same procedure was performed after the second block (364.4 mg BuOx; 2.87 mmol, 20 eq, 10% w/w analyzed). Block three (306.9 mg MeOx; 3.6 mmol, 28 eq) was added, the polymerization was terminated using 80 µL piperidine and the product was obtained as a colorless solid (598 mg, 65%, Mₜₕ = 6.6 kg/mol).
GPC (DMAc): Mₙ = 9.9 kg/mol (PDI 1.2₃); ¹H-NMR (CDCl₃, 298 K): δ = 3.45 (br, 405H, (N-CH₂C*H*₂)); 3.03/2.95 (m, 3H, N-C*H*₃^{Ini}); 2.43 -1.86 (m, 329H, CO-C*H*₃, CO-C*H*₂, C*H*₂^{Pid}); 1.57 (br, 63H, CH₂-C*H*₂-CH₂-); 1.32 (br, 60H, -C*H*₂-CH₃); 0.91 ppm (br, 88H, -C*H*₃^{butyl}), Mₙ = 10.0 kg/mol (MeOx₃₆-b-BuOx₃₀-b-MeOx₃₆).

### Preparation of Methyl-P[EtOx₅₀-b-BuOx₁₉]-piperazine (P4)

P4 was prepared accordingly from 10 mg MeOTf (61 µmol, 1 eq), 321 mg 2-ethyl-2-oxazoline (EtOx, 3.24 mmol, 53 eq, 1^{st} block) and 157 mg BuOx (1.23 mmol, 20 eq, 2^{nd} block), using 150 mg piperazine as a terminating reagent. For precipitation, a solvent mixture of cyclohexane and diethylether (50/50, v/v) was used. The product was obtained as a colorless solid (yield 0.36 g, 77 %, Mtₕ = 7.8 kg/mol).
GPC (DMAc): Mₙ = 11.5 kg/mol (PDI 1.0₉); ¹H-NMR (CDCl₃, 298 K): δ = 3.45 (br, 276H, (N-CH₂C*H*₂)); 3.04/2.95 (m, 3H, N-C*H*₃^{Ini}); 2.5-2.2 (m, 144H, CO-C*H*₂-CH₃, CO-C*H*₂, C*H*₂^{Pid}); 1.58 (br, 37H, CH₂-C*H*₂-CH₂-); 1.34 (br, 41H, -C*H*₂-CH₃); 1.11 (br, 151H, CO-CH₂-C*H*₃); 0.91 ppm (br, 56H, -C*H*₃^{butyl}), Mₙ = 7.5 kg/mol (EtO*x*₅₀-b-BuOx₁₉).

### 3. Pyrene Fluorescence measurements

The CMC was determined using standard procedure. In short, a pyrene solution in acetone (2.5 mM) was added to vials and the solvent was allowed to evaporate. Polymer solutions at appropriate concentrations in assay buffer were added to the vials so that a final concentration of 5 × 10⁻⁷ M of pyrene was obtained. The solutions were incubated at 25 °C (>2 h) and the pyrene fluorescence spectrum were recorded using a Fluorolog3 (HoribaJobinYvon) λₑₓ = 333 nm, λₑₘ = 360-400 nm, slidwidth(ex) = slidwidth(em) = 1 nm, step width 0.5 nm. Typically, five spectra of each data point were averaged (integration time 0.1 s, if necessary 10 spectra with 0.2 s integration), the CMC is assumed where a steep increase in fluorescence intensity is observed. Furthermore, the fluorescence intensity of the I₁ band was compared to the intensity of I₃ band which gives an estimate of the polarity of the environment of the pyrene probe. No excimer band formation was observed.

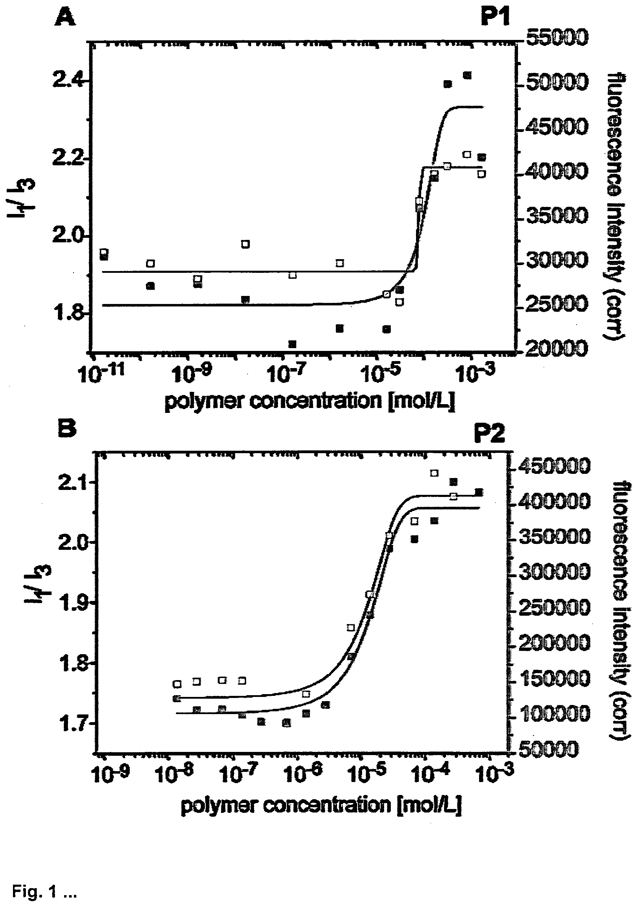

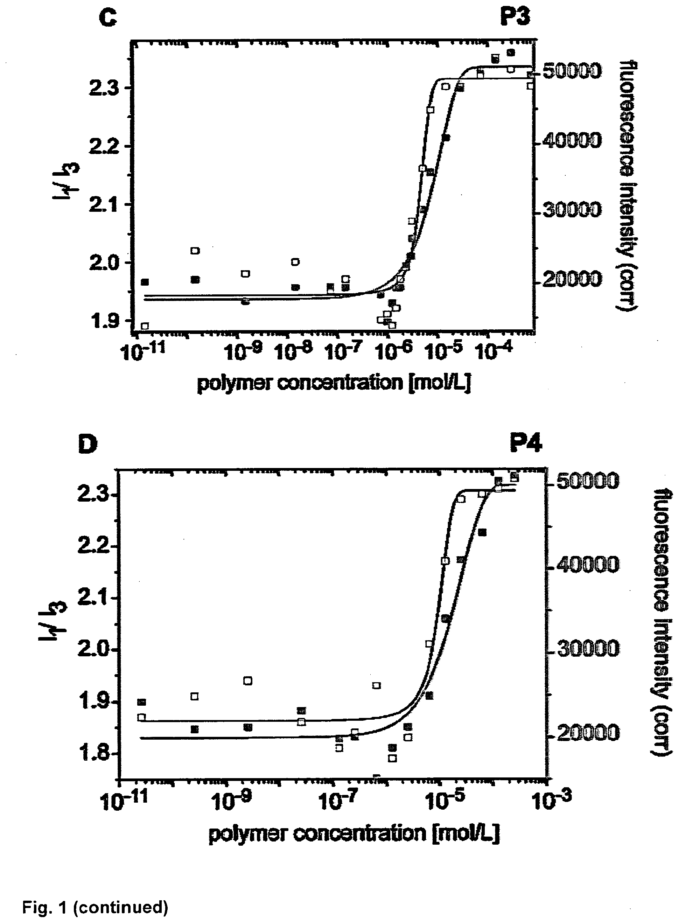

Using this approach, we found low critical micelle concentrations (CMC) ranging from 100 mg/L (15 µM, P1), 20 mg/L (2.7 µM, P2), 7 mg/L (1 µM, P3) to 6 mg/L (0.7 µM, P4), respectively (cf. Figure 1).

Figure 1 shows the fluorescence intensity and I₁/I₃ ratios of pyrene solutions (5 × 10-7 M in phosphate buffered saline (PBS)) in dependence of concentration of block copolymers as used in the context of the invention at 25 °C.

### 4. Drug solubilization studies

### 4.1 Paclitaxel (PTX) solubilization

Drug-polymer solutions were prepared using the thin film method. Appropriate amounts of polymer and PTX (Sigma-Aldrich, St. Louis, MO, order number T7191) (stock solution 5-8 mg/mL in ACN or ethanol) were solubilized in minimum amounts of ACN or ethanol, alternatively. After complete removal of the solvent, the solid polymer-drug film was taken up in assay buffer or deionized water. Exemplary, a procedure is given as follows.

The solvent was removed in a stream of air under mild warming and the films were subjected to 0.2 mbar for at least 3 hours to remove eventually present residual solvent. Subsequently 200 µL of assay buffer (aqueous solution, containing 122 mM NaCl, 25 mM Na₂CO₃, 10 mM HEPES, 10 mM Glucose, 3 mM KCI, CaCl₂ 1.4 mM and K₂HPO₄ 0.4 mM, pH = 7.4) were added to obtain final polymer concentration. At higher PTX concentration solubilization was facilitated by incubation of the solutions for 50 - 60 °C for typically 5-10 min. The clear solutions were filtered through HPLC syringe filters (0.45 µm pore size) and subjected to HPLC analysis (vide infra). Substitution of the relatively toxic ACN with the more benign EtOH as a common solvent before film formation did not diminish loading efficiencies.

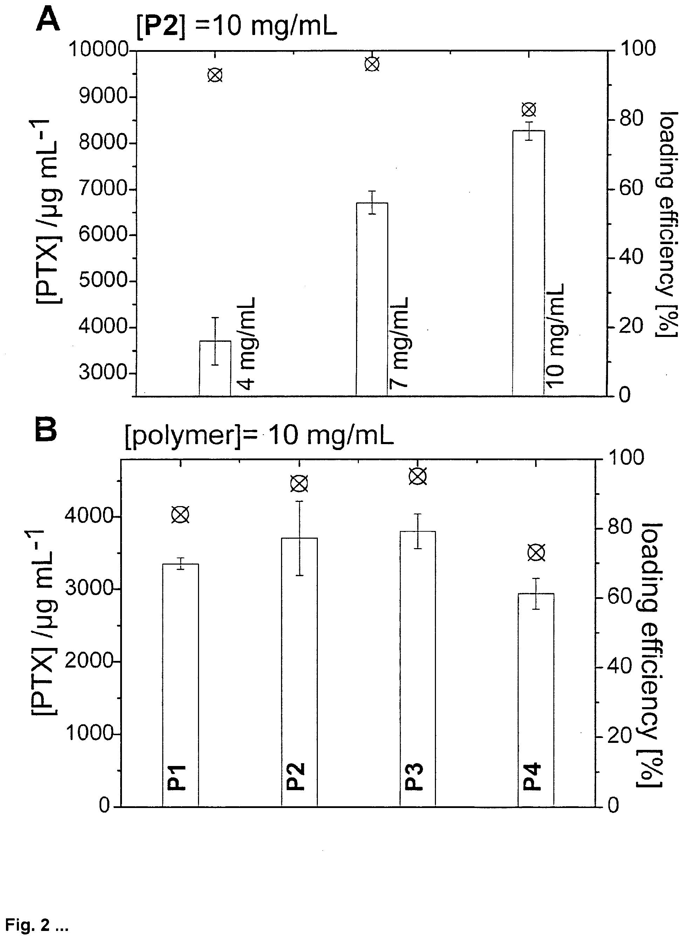

It was attempted to solubilize 4, 7 and 10 mg/mL PTX with 10 mg/mL of P2. Up to concentrations of 7 mg/mL PTX, clear solutions were obtained after mild heating (approx. 40 °C) for a short time. Under these conditions, the solubilization of PTX was complete as confirmed by high performance liquid chromatography (HPLC) (cf. Figure 2A).

Only at 10 mg/mL PTX some clear crystals remained undissolved even after 30 min heating at 60 °C. However, still extraordinary amounts of 8.2 mg/mL of PTX were solubilized and found in the aqueous phase, in other words, the resulting formulation consists of at least 40 % wt. PTX. Similar results were obtained with the other polymers including P1, having only 12 units in the BuOx block (cf. Figure 2B).

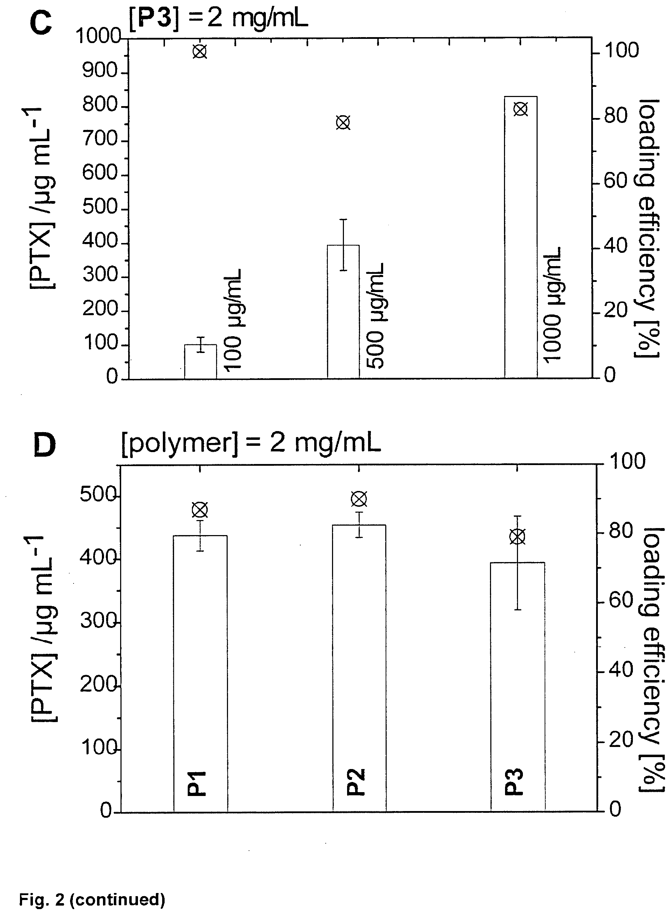

Even at polymer concentration as low as 2 mg/mL, excellent loading efficiencies and total drug loading of about 30% wt. were obtained (cf. Figure 2C, D). Thus, the block copolymers used in the context of the invention can reduce the amount of excipient needed to solubilize PTX by approx. 100 and 9 times, as compared to Cremophor EL/ethanol (CrEL) and Abraxane™, respectively.

Figure 2 shows the results of the solubilization of PTX with amphiphilic block copolymers using the film method. A-D) Solution concentration of PTX (bars) and loading efficiency (crossed circles) using different polymers and targeted PTX concentrations: A) P2 (10 mg/mL) and 4 mg/mL, 7 mg/mL and 10 mg/mL PTX. B) P1-P4 (10 mg/mL) and 4 mg/mL PTX; C) P3 (2 mg/mL) and 100 µg/mL, 500 µg/mL and 1 mg/mL PTX; D) P1-P3 (2 mg/mL) and 500 µg/mL PTX.

Data are presented as means (n=3; except for C: 1 mg/mL PTX n=1, and B: P4 n =2) ± SEM.

### 4.2 Solubilization of Cyclosporin A

Solubilization of Cyclosporin A (Alexis Corporation San Diego, CA, order number 380-002-G001) was performed accordingly using the film method. Using P2 and Cyclosporin A clear and stable solutions were obtained.

### 4.3 Solubilization of Amphotericin B

Solubilization of amphotericin B with P2 was carried out using solvent exchange by dialysis. P2 (10.2 mg) and amphotericin B trihydrate (2.1 mg, Riedel-de Haën, Seelze, Germany, order number 46006) were dissolved in 250 µL dimethylsulfoxide (DMSO) to yield a clear, yellow solution. A total of 750 µL of deionized water was added, after 100µL the mixture became turbid. The resulting mixture was transferred into a dialysis bag (MWCO 3500 g/mol). The solution was dialyzed against 2 L deionized water (water exchanged at 2 h, 4 h and 22 h). After a total of 50 h, the suspension (4 mL) was recovered from the bag. An aliquot of 500 µL was filtered (0.45 µm) to remove particles and the clear, yellow solution was freeze dried to yield 1 mg of yellow foam-like solid. The residue was dissolved in 200 µL DMSO and the amphotericin B was quantified spectrophotometrically using the absorbance at 410 nm. The dialyzed solution contained 366 µg Amphotericin B (18 % (w/w) with respect to P2). Another aliquot of 1 mL was freeze-dried (2.2 mg yellow foam) and dissolved subsequently in 100 µL deionized water. The polymer-drug foam dissolved rapidly and completely to give an intense yellow solution of low viscosity. Thus, without the need for cryoprotectants, 3.7 mg/mL of Amphotericin B could be solubilized using only 18.3 mg/mL P2. Using the same protocol, water solubility of Amphotericin B was determined to be approx. 0.4 µg/mL.

### 4.4 HPLC analysis of drug solubilization

HPLC analysis was carried out under isocratic conditions using a Shimadzu system comprising a SCL-10A system controller, SIL-10A autoinjector, SPD-10AV UV detector and two LC-10 AT pumps. As stationary phase a Nucleosil C18-5µ column was used (250 mm x 4 mm), as a mobile phase an acetonitrile/water mixture (55/45, v/v) was applied. Detection was performed at 220 nm. The amount of PTX in the polymer solution was calculated using a calibration curve obtained with known amounts of PTX dissolved in acetonitrile and analyzed accordingly.

HPLC analysis of Cyclosporin A solutions obtained using the protocol described above were performed using as a mobile phase an acetonitrile/water mixture (90/10, v/v) at 70 °C. With 5 mg/mL of P2, 1.03 mg/mL of Cyclosporin A could be solubilized. This corresponded to an 82 % loading efficiency and 17 % loading (w/w). Thus, an approx. 120 fold increase of Cyclosporin A solubility was achieved using P2.

### 4.5 Micelle Characterization

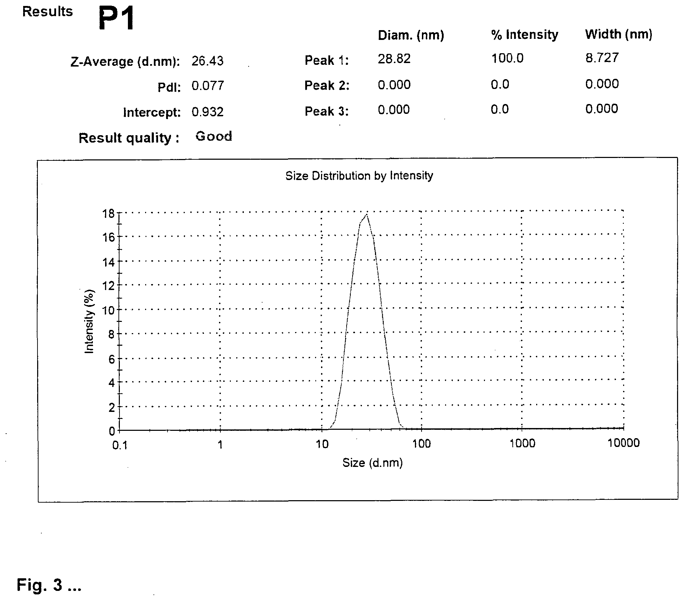

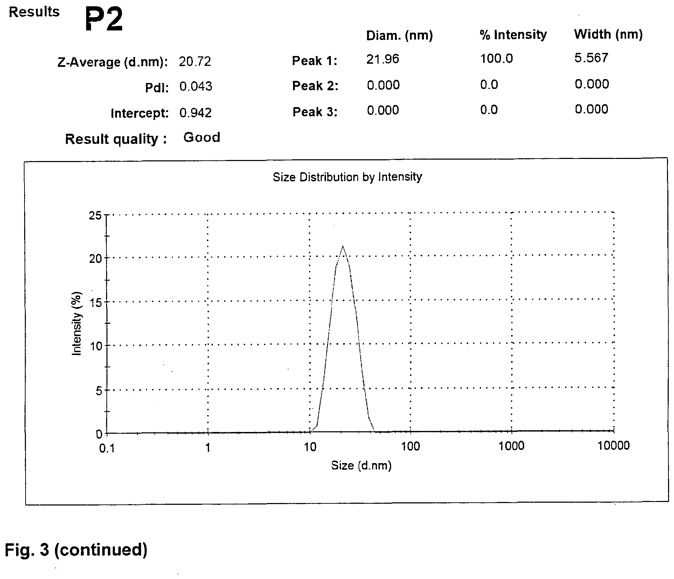

The drug loaded micelles found to be very small (rh = 12-22 nm) and show a narrow size distribution (PD = 0.04 - 0.12) as determined by dynamic light scattering. Figure 3 shows the results of dynamic light scattering measurements on drug loaded micelles of P1 and P2 (10 mg/mL), each 4mg/mL PTX.

### 5. Cytotoxicity assay: MTT

MCF7/ADR cells (derived from human breast carcinoma cell line, MCF7 (ATCC HT-B22) by selection with Doxorubicin, was kindly presented by Y.L. Lee (William Beaumont Hospital, Royal Oak, MI). Cells were maintained in Dulbecco's Modified Eagle's Medium (DMEM), containing 10% heat inactivated fetal bovine serum (FBS) and 1% penicillin/streptomycin as described elsewhere. All tissue material media was obtained from Gibco Life Technologies, Inc. (Grand Island, NY).

MCF7/ADR were seeded in 96 well plates (10⁴ cells per well) and were allowed to reattach for 24h. Treatment solutions were prepared from a 1 mg/mL polymer stock solution in assay buffer (containing 122 mM NaCI, 25 mM NaHCO₃, 10 mM glucose, 10 mM HEPES, 3 mM KCI, 1.2 mM MgSO₄, 1.4 mM CaCl₂, and 0.4 mM K₂HPO₄, pH 7.4) by appropriate dilution with media (Dulbecco's Modified Eagle's Medium (DMEM), supplemented with 10% FBS, 25 mM HEPES and penicillin/streptomycin). The cells were incubated for 48 h with 200 µL of treatment solution. After discarding the treatment solution, cells were washed thrice with PBS. FBS-free DMEM (100 µL/well) as well as 25 µL of a 5 mg/mL solution of 3-(4,5-dimethylthiazol-2-yl)-2,5-diphenyltetrazolium bromide (MTT, Invitrogen, Eugene, OR, USA) in PBS were added and the cells incubated at 37 °C for 2 hours. The media was discarded subsequently and replaced with 100 µL of solvent (25% v/v DMF, 20% w/v SDS in H₂O). The purple formazan product was allowed to dissolve over night and the absorbance at 570 nm was obtained using a plate reader (SpectraMax M5, Molecular Devices). Positive controls were cells treated with media alone; negative controls were wells without cells. Each concentration was repeated in four wells, results are expressed as mean ± SEM.

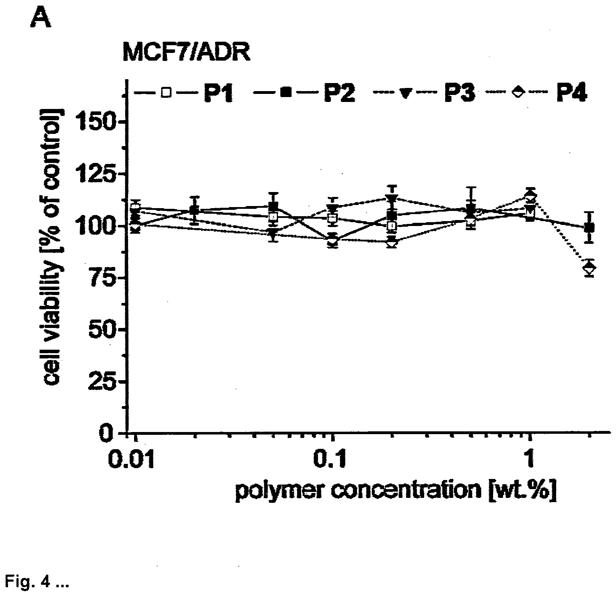

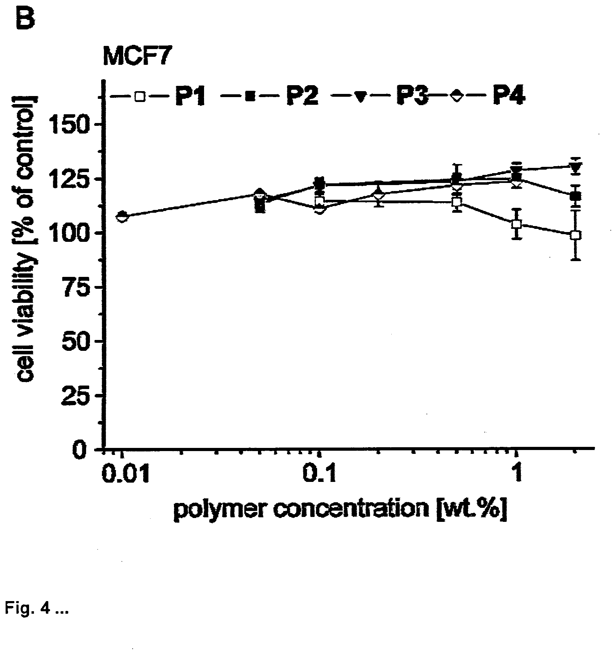

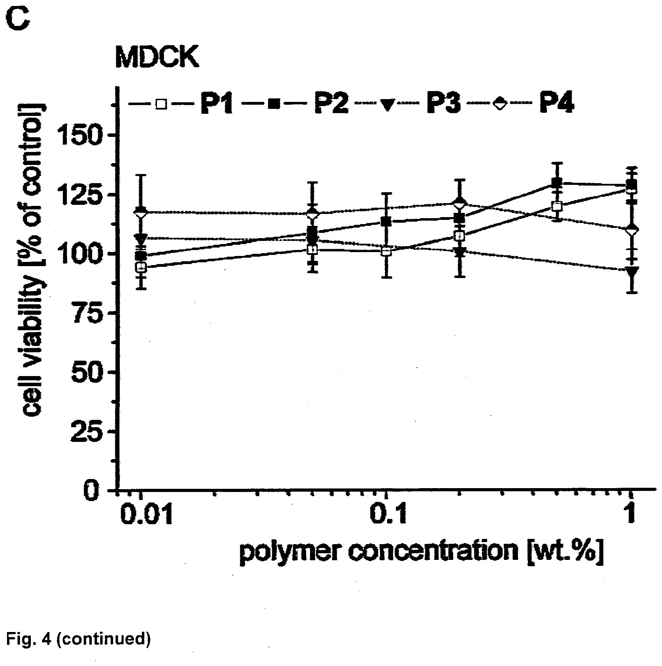

Figure 4 shows the results of the assays for cytotoxicity of polymers P1-P4 in multi-drug resistant (MCF7/ADR, A) and non-resistant (MCF7, B) human adenocarcinoma cells and Madin-Darby canine kidney (MDCK) cells after incubation for 24 h (A) and 2 h (B,C). Data expressed as mean ± SEM (n = 4). P1-P4 alone were found to be non-cytotoxic at concentrations of up to 20 mg/mL and for 24 h incubation using different cell lines.

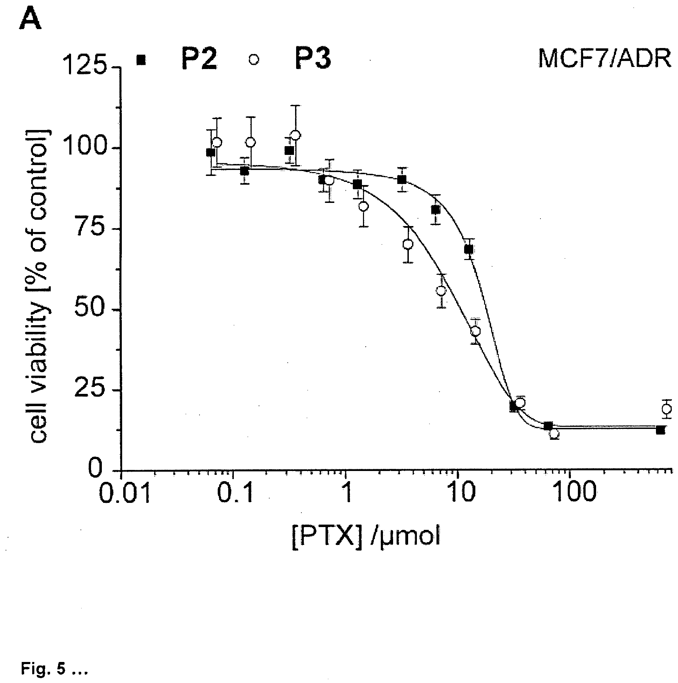

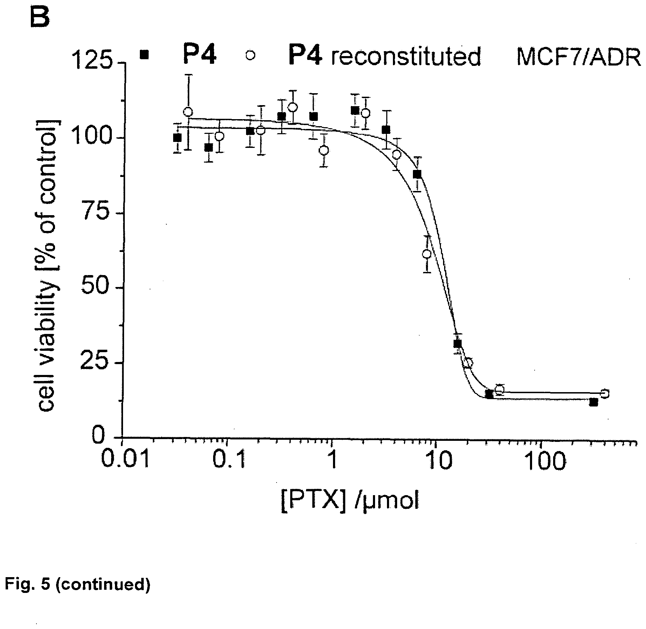

In contrast to the plain polymers, the PTX-loaded micelles displayed a pronounced, concentration-dependent toxicity with respect to human and murine tumor cell lines (Figure 5A). For example, after 24 h incubation with PTX-loaded P2, P3 and P4, IC₅₀ values in the range of 10µM were observed using the resistant cell line (MCF7/ADR). Commercially available CrEL-PTX formulation was used as a control and resulted in comparable growth inhibition. The PTX-loaded micelles could be lyophilized without the need for cryoprotectants and simply be redispersed in water or saline without compromising the drug loading, the particle size or the in vitro drug efficacy (Figure 5B).

Figure 5 shows the paclitaxel dose dependent viability of multi-drug resistant MCF7/ADR cells. A) Comparison of P2 and P3 formulated PTX shows no marked difference for the cell viability in dependence of the carrier material after 24h of incubation. B) Exemplified for P4, no change in paclitaxel activity is observed after freeze-drying and reconstitution in deionized water. Data is presented as means (n=3) ± SEM.

### 6. Animal studies

All experiments were performed using female C57/BI/6 mice 11-12 weeks of age (Taconic Laboratories, Germantown, NY). The animals were kept five per cage with an air filter cover under light (12-h light/dark cycle) and temperature-controlled (22F1 8C) environment. All manipulations with the animals were performed under a sterilized laminar hood. Food and water were given ad libitum. The animals were treated in accordance to the Principles of Animal Care outlined by National Institutes of Health, and protocols were approved by the Institutional Animal Care and Use Committee of the University of Nebraska Medical Center. Lewis lung carcinoma cells (LLC 3T) were grown in T75 flasks and collected with HBSS. Cell suspensions (1 × 10⁶ per animal) were injected subcutaneously in a volume of 50 µL on the right flank. After tumors appeared tumor sizes where recorded (day 1) and treatment solutions were injected at a doses of 10 mg/kg PTX in a volume of 100 µL on day 1, 4 and 7.

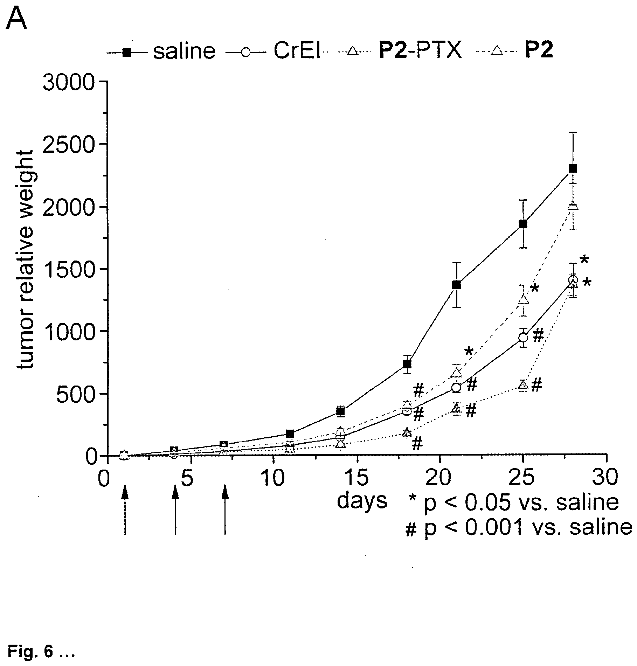

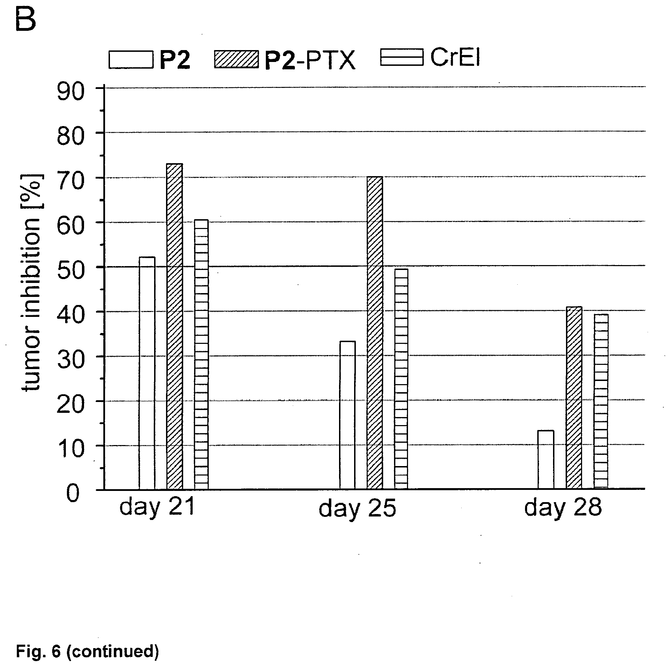

The in vivo anti-tumor effect of PTX-loaded micelles was examined in C57/BI/6 mice with subcutaneous Lewis Lung carcinoma tumors (Figure 6). Both commercial (CrEI) and reconstituted poly(2-oxazoline) formulations (P2-PTX) significantly (p < 0.05) decreased tumor burden after only one injection (day 4, tumor inhibition = 72% and 63 %, respectively). The tumors in the P2-PTX treated animals remained significantly smaller (p < 0.05) than in the animals treated with the commercial product between days 11 and 25. We found the tumor inhibition by P2-PTX in this period to be approximately 70 % as compared to 50-60 % in the CrEI group. After 28 days, however, a sharp increase in the tumor burden of the animals in the P2-PTX regimen was observed and the same tumor inhibition in both treated groups was found.

Figure 6 A) shows relative tumor weights of subcutaneous Lewis Lung carcinoma tumors in C57/BI/6 mice comparing negative controls (saline, P2 alone), treatment with POx solubilized PTX (P2-PTX) and commercial product (CrEI) at the same PTX doses (10 mg/kg). Arrows indicate times of injection. B) Tumor inihibition of P2, P2-PTX and commercial CrEI. Data represented as means ± SEM (n=5). Figure 6 B) shows the calculated tumor inhibition in treatment groups of P2, P2-PTX and CrEI at different points of time.

Also disclosed:
1. A composition, comprising:
   (a) at least one copolymer comprising repeating units of formula (I) 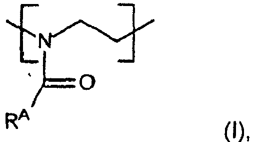
      wherein R^{A} is a hydrocarbon group, optionally substituted with -OH, -SH, -COOH, - NR'₂, -COOR', -CONR', -CHO, with R' representing H or C₁₋₃ alkyl, and with R^{A} being selected such that the repeating unit of formula (I) is hydrophilic;
      and repeating units of the formula (II), 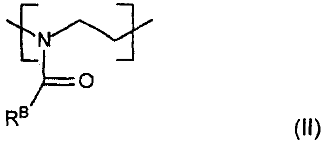 wherein R^{B} is a hydrocarbon group optionally substituted with halogen, -OH, -SH, - COOH, -NR"₂, -COOR", -CONR", -CHO, with R" representing H, alkyl or alkenyl, and with R^{B} being selected such that the repeating unit of formula (II) is more hydrophobic than the repeating unit of formula (I); and
   (b) one or more active agent(s).
2. Composition according to item 1, wherein R^{A} is selected, independently for each occurrence, from methyl and ethyl optionally substituted with -OH, -SH, -COOH, -NR'₂, -COOR', -CONR', -CHO, with R' representing H or C₁₋₃ alkyl and wherein R^{B} is selected from a C₃₋₂₀ hydrocarbon group.
3. Composition according to item 1, wherein R^{A} is selected, independently for each occurrence, from methyl and ethyl and R^{B} is selected from C₄₋₆ alkyl.
4. Composition according to any of items 1 to 3, wherein the copolymer is a block copolymer comprising at least one poly(2-oxazoline) block A consisting of repeating units of formula (I) as defined in any of claims 1 to 3, and at least one poly(2-oxazoline) block B consisting of repeating units of formula (II) as defined in any of items 1 to 3.
5. Composition according to item 4, wherein at least one block A is a poly(2-methyl-2-oxazoline) or a poly(2-ethyl-2-oxazoline) block and at least one block B is a poly(2-butyl-2-oxazoline) block.
6. Composition according to a item 4 or 5, wherein the block copolymer is an AB or BA diblock copolymer or a ABA or BAB triblock copolymer.
7. Composition according to any of items 1 to 6, wherein at least one active agent is a hydrophobic active agent.
8. Composition according to any of items 1 to 7, wherein the at least one copolymer forms micelles incorporating the active agent.
9. Composition according to any of items 1 to 8, wherein the weight ratio of active agent(s) to copolymer(s) ranges from 1:10 to 1:1.
10. Composition according to any of items 8 or 9, wherein the micelles have a size of 5 to 500 nm.
11. Composition according to any of items 1 to 10, wherein the active load, expressed as the ratio of the weight of the active agent or drug to the sum of the weights of the active agent and the block copolymer, of 10 % or more.
12. Composition according to any of items 1 to 11 which is a pharmaceutical or diagnostic composition containing a drug as an active agent.
13. A copolymer comprising repeating units of formula (I) as defined above wherein R^{A} is selected from methyl and ethyl and repeating units of the formula (II) wherein R^{B} is butyl.
14. A copolymer according to item 13, which is a block copolymer comprising at least one block A selected from poly(2-methyl-2-oxazoline) and poly(2-ethyl-2-oxazoline) and at least one block B selected from poly(2-(C₄₋₆)alkyl-2-oxazoline).
15. Use of a composition according to any of items 1 to 11 as a plant protecting composition.
16. Use of a copolymer as defined in any of items 1 to 6, 13 or 14 for the solubilisation of an active agent in water or an aqueous solution.
17. A method for the detection of active compounds which interact with a target of interest in a screening test, including the steps of incorporating an active compound into a composition as defined in any of items 1 to 11 and subjecting the composition to the screening test.
18. A method for treating or preventing a disorder or disease, the method comprising the administration of the pharmaceutical composition of item 12 to a subject in need of such a treatment or prevention.

## Claims

1. A pharmaceutical or diagnostic composition, comprising:
(a) at least one block copolymer comprising at least one poly(2-oxazoline) block A consisting of repeating units of formula (I) 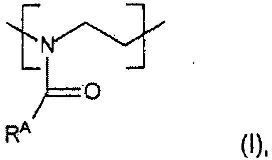
wherein R^{A} is a hydrocarbon group, optionally substituted with -OH, -SH, -COOH, -NR'₂, -COOR', -CONR', -CHO, with R' representing H or C₁₋₃ alkyl, and with R^{A} being selected such that the repeating unit of formula (I) is hydrophilic;
and at least one poly(2-oxazoline) block B consisting of repeating units of formula (II) 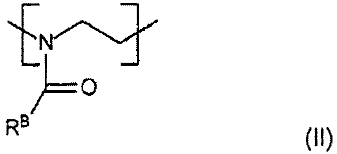 wherein R^{B} is a hydrocarbon group optionally substituted with halogen, -OH, -SH, -COOH, -NR"₂, -COOR", -CONR", -CHO, with R" representing H, alkyl or alkenyl, and with R^{B} being selected such that the repeating unit of formula (II) is more hydrophobic than the repeating unit of formula (I); and
(b) one or more drugs as hydrophobic active agent(s).

2. The pharmaceutical or diagnostic composition according to claim 1, wherein R^{A} is selected, independently for each occurrence, from methyl and ethyl optionally substituted with -OH, -SH, -COOH, -NR'₂, -COOR', -CONR', -CHO, with R' representing H or C₁₋₃ alkyl and wherein R^{B} is selected from a C₃₋₂₀ hydrocarbon group.

3. The pharmaceutical or diagnostic composition according to claim 1, wherein R^{A} is selected, independently for each occurrence, from methyl and ethyl and R^{B} is selected from C₄₋₆ alkyl.

4. The pharmaceutical or diagnostic composition according to any of claims 1 to 3, wherein at least one block A is a poly(2-methyl-2-oxazoline) or a poly(2-ethyl-2-oxazoline) block and at least one block B is a poly(2-butyl-2-oxazoline) block.

5. The pharmaceutical or diagnostic composition according to any of claim 1 to 4, wherein the block copolymer is an AB or BA diblock copolymer or a ABA or BAB triblock copolymer.

6. Composition according to any of claims 1 to 5, wherein the at least one copolymer forms micelles incorporating the active agent.

7. Composition according to any of claims 1 to 6, wherein the weight ratio of active agent(s) to copolymer(s) ranges from 1:10 to 1:1.

8. Composition according to any of claims 6 or 7, wherein the micelles have a size of 5 to 500 nm.

9. Composition according to any of claims 1 to 8, wherein the active load, expressed as the ratio of the weight of the active agent to the sum of the weights of the active agent and the block copolymer, is 10 % or more.

## Patentansprüche

1. Eine pharmazeutische oder diagnostische Zusammensetzung, umfassend:
(a) mindestens ein Blockcopolymer, umfassend mindestens einen Poly(2-oxazolin)-Block A, bestehend aus Wiederholungseinheiten der Formel (I) 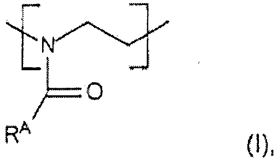
wobei R^{A} eine Kohlenwasserstoffgruppe ist, gegebenenfalls substituiert mit -OH, -SH, -COOH, -NR'₂, -COOR', -CONR', -CHO, wobei R' H oder C₁₋₃-Alkyl darstellt, und wobei R^{A} so ausgewählt ist, dass die Wiederholungseinheit der Formel (I) hydrophil ist;
und mindestens einen Poly(2-oxazolin)-Block B, bestehend aus Wiederholungseinheiten der Formel (II) 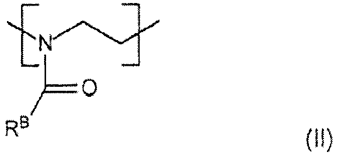 wobei R^{B} eine Kohlenwasserstoffgruppe ist, gegebenenfalls substituiert mit Halogen, -OH, -SH, -COOH, -NR"₂, -COOR", -CONR", -CHO, wobei R" H, Alkyl oder Alkenyl darstellt, und wobei R^{B} so ausgewählt ist, dass die Wiederholungseinheit der Formel (II) hydrophober als die Wiederholungseinheit der Formel (I) ist; und
(b) einen oder mehrere Arzneistoff(e) als hydrophobe(n) Wirkstoff(e).

2. Die pharmazeutische oder diagnostische Zusammensetzung nach Anspruch 1, wobei R^{A}, unabhängig für jedes Auftreten, ausgewählt ist aus Methyl und Ethyl, gegebenenfalls substituiert mit -OH, -SH, -COOH, -NR'₂, -COOR', -CONR', -CHO, wobei R' H oder C₁₋₃-Alkyl darstellt und wobei R^{B} aus einer C₃₋₂₀-Kohlenwasserstoffgruppe ausgewählt ist.

3. Die pharmazeutische oder diagnostische Zusammensetzung nach Anspruch 1, wobei R^{A}, unabhängig für jedes Auftreten, aus Methyl und Ethyl ausgewählt ist und R^{B} aus C₄₋₆-Alkyl ausgewählt ist.

4. Die pharmazeutische oder diagnostische Zusammensetzung nach einem der Ansprüche 1 bis 3, wobei mindestens ein Block A ein Poly(2-methyl-2-oxazolin)- oder ein Poly(2-ethyl-2-oxazolin)-Block ist und mindestens ein Block B ein Poly(2-butyl-2-oxazolin)-Block ist.

5. Die pharmazeutische oder diagnostische Zusammensetzung nach einem der Ansprüche 1 bis 4, wobei das Blockcopolymer ein AB- oder BA-Diblockcopolymer oder ein ABA- oder BAB-Triblockcopolymer ist.

6. Zusammensetzung nach einem der Ansprüche 1 bis 5, wobei das mindestens eine Copolymer Mizellen bildet, die den Wirkstoff beinhalten.

7. Zusammensetzung nach einem der Ansprüche 1 bis 6, wobei das Gewichtsverhältnis von Wirkstoff(en) zu Copolymer(en) im Bereich von 1:10 bis 1:1 liegt.

8. Zusammensetzung nach einem der Ansprüche 6 oder 7, wobei die Mizellen eine Größe von 5 bis 500 nm aufweisen.

9. Zusammensetzung nach einem der Ansprüche 1 bis 8, wobei die Wirkstoffbeladung, ausgedrückt als das Verhältnis des Gewichts des Wirkstoffs zur Summe der Gewichte des Wirkstoffs und des Blockcopolymers, 10% oder mehr beträgt.

## Revendications

1. Composition pharmaceutique ou diagnostique, comprenant :
(a) au moins un copolymère à blocs comprenant au moins un bloc poly(2-oxazoline) A constitué par des motifs répétitifs de formule (I) 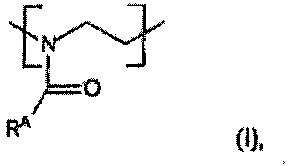
dans laquelle R^{A} est un groupe hydrocarboné, éventuellement substitué par -OH, -SH, -COOH, -NR'₂, -COOR', -CONR', -CHO, où R' représente H ou un alkyle en C₁₋₃, et R^{A} est choisi de sorte que le motif répétitif de formule (I) soit hydrophile ;
et au moins un bloc poly(2-oxazoline) B constitué par des motifs répétitifs de formule (II) 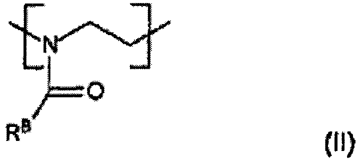 dans laquelle R^{B} est un groupe hydrocarboné éventuellement substitué par un halogène, -OH, -SH, -COOH, -NR"₂, -COOR", -CONR", -CHO, où R" représente H, un alkyle ou alcényle, et R^{B} est choisi de sorte que le motif répétitif de formule (II) soit plus hydrophobe que le motif répétitif de formule (I) ; et
(b) un ou plusieurs médicaments à titre de principe(s) actif(s) hydrophobe(s).

2. Composition pharmaceutique ou diagnostique selon la revendication 1, dans laquelle R^{A} est choisi, indépendamment à chaque occurrence, parmi un méthyle et éthyle éventuellement substitué par -OH, -SH, -COOH, -NR'₂, -COOR', -CONR', -CHO, où R' représente H ou un alkyle en C₁₋₃ et dans laquelle R^{B} est choisi parmi un groupe hydrocarboné en C₃₋₂₀.

3. Composition pharmaceutique ou diagnostique selon la revendication 1, dans laquelle R^{A} est choisi, indépendamment à chaque occurrence, parmi un méthyle et éthyle et R^{B} est choisi parmi un alkyle en C₄₋₆.

4. Composition pharmaceutique ou diagnostique selon l'une quelconque des revendications 1 à 3, dans laquelle au moins un bloc A est un bloc poly(2-méthyl-2-oxazoline) ou poly(2-éthyl-2-oxazoline) et au moins un bloc B est un bloc poly(2-butyl-2-oxazoline).

5. Composition pharmaceutique ou diagnostique selon l'une quelconque des revendications 1 à 4, dans laquelle le copolymère à blocs est un copolymère dibloc AB ou BA ou un copolymère tribloc ABA ou BAB.

6. Composition selon l'une quelconque des revendications 1 à 5, dans laquelle ledit au moins un copolymère forme des micelles renfermant le principe actif.

7. Composition selon l'une quelconque des revendications 1 à 6, dans laquelle le rapport en poids du ou des principes actifs au(x) copolymère(s) est dans la plage de 1:10 à 1:1.

8. Composition selon l'une quelconque des revendications 6 ou 7, dans laquelle les micelles ont une taille de 5 à 500 nm.

9. Composition selon l'une quelconque des revendications 1 à 8, dans laquelle la charge active, exprimée sous forme de rapport du poids du principe actif à la somme des poids du principe actif et du copolymère à blocs, est de 10 % ou plus.
